**Europäisches Patentamt**

**European Patent Office**

**Office européen des brevets**

(19)

(11) **EP 1 569 018 A2**

(12) # EUROPEAN PATENT APPLICATION

(43) Date of publication:
**31.08.2005 Bulletin 2005/35**

(51) Int Cl.[7]: **G02B 6/122**, G02B 6/255,
G02B 6/30, C09J 5/02

(21) Application number: **05101178.1**

(22) Date of filing: **17.02.2005**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **17.02.2004 JP 2004039970**

(71) Applicant: **OMRON CORPORATION**
**Kyoto 600-8530 (JP)**

(72) Inventors:
• **Yoshitake, Naoki**
**600-8530, Kyoto (JP)**
• **Hosokawa, Hayami**
**600-8530 JAPAN, KYOTO (JP)**
• **Yasuda, Naru**
**600-8530 JAPAN, KYOTO (JP)**
• **Takahashi, Toshiyuki**
**600-8530 JAPAN, Kyoto (JP)**

(74) Representative: **Weihs, Bruno Konrad et al**
**Osha & May**
**121, avenue des Champs Elysées**
**75008 Paris (FR)**

(54) **Waveguide equipment and polymer waveguide**

(57)    An oxide film 27 is formed on an end surface of a waveguide 21 which includes an under cladding 24, a core 25 and a over cladding 26. An end surface of a fiber guide 22, 23 holding an optical fiber 32, 36 is coupled the end surface of the waveguide 21 formed the oxide film 27 with a glue. It is preferable that the oxide film is $SiO_x$ ($1 \leqq x \leqq 1.5$). This oxide film 27 has a composition ratio of an oxygen atom smaller than the stable composition, and thus it is easy for OH radicals to appear on the surface of the oxide film. Such OH radicals bond chemically with the resin of the waveguide 21 and the glue 37. Thus, an adhesive strength between the waveguide 21 and the fiber guide 22, 23 is improved.

[FIG. 5]

EP 1 569 018 A2

**Description**

BACKGROUND OF INVENTION

Field of the Invention

The invention relates generally to waveguide equipment and a polymer waveguide for optical communications.

Background Art

In a connected portion or an end portion of an optical fiber cable for optical communications, waveguide equipment is used to connect the end of one optical fiber cable to, for example, another optical fiber cable, a light projection device, a photo detector, etc. So, it is requested that such waveguide equipment can be produced with low cost and be suitable for a mass production, as the use of the optical communication, which can transmit large capacity data with high-speed, has increased in recent years.

As a result, a polymer waveguide using a high molecular compound (polymer) is suggested for the waveguide. When the waveguide equipment is assembled by integrating the polymer waveguide with the optical fiber made of glass or polymer, an end surface of the polymer waveguide is integrated with an end surface of the fiber guide which holds the optical fiber using high molecular glue.

The optical fiber or the optical fiber arrays are made of silica glass. So, adhesion is high between the glue and the optical fiber or the optical fiber arrays. This is because glass has many OH radicals and has high affinity with glue; so the glue is spread on the surface of the glass and bonds with the OH radicals on the surface of glass by hydrogen bond or van der Waals forces. And, if UV hardening glue is applied on a silane coupling agent on the glass, adhesion can be improved by a chemical bond. But, adhesion between the polymer waveguide and the glue is not stronger than that between the glass and the glue. Because most of the association of each atom in highly-polymer compounds are connected by hardening entirely, and there are few OH radicals on the surface of the waveguide to bond with the glue. So, the hydrogen bonding strength, the van der Waals forces, and the chemical bonding strength become weak. Moreover, unevenness watched with a numerator level appears to an end surface of the waveguide, and all of the OH radicals expressed in an end surface of the waveguide do not bond with glue. So, this adhesive strength between the polymer waveguide and the fiber guide is weak, and it is easy to exfoliate by high temperature and high air moisture. Thus, there is a problem in the reliability of the adhesive strength.

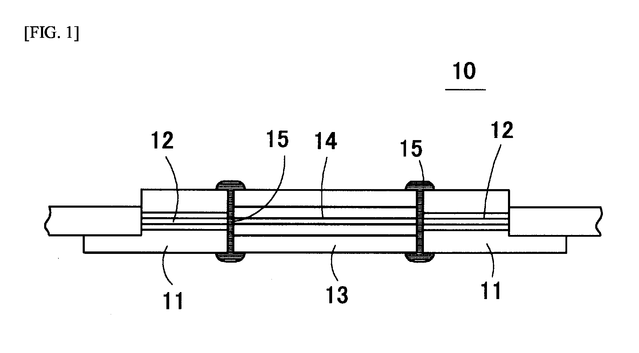

FIG. 1 is a schematic cross-sectional view of the prior art that improved reliability of the adhesive strength. In this waveguide equipment 10, an optical fiber 12 is held to an optical fiber guide 11 and couples to a core 14 in a waveguide 13 optically. An end of the fiber guide 11 bonds with an end surface of the waveguide 13 using glue 15. Moreover, the glue 15 is applied to an outer peripheral portion of the bonding surface between the fiber guide 11 and the waveguide 13, and it is hardened. As a result, exfoliation at the bonding surface is prevented between the fiber guide 11 and the waveguide 13. In addition, $SiO_2$ film is disposed on the surface of the glue 15 applied to the outer peripheral portion of the bonding surface, so an invasion of the moisture is prevented to the adhesive surface.

However, degradation of adhesive strength is only prevented by preventing the invasion of moisture and the exfoliation from the outer peripheral portion of the bonding surface. These methods could not improve adhesive strength with the waveguide and the optical fiber fundamentally.

SUMMARY OF INVENTION

Embodiments of the present invention improve the adhesive strength between the polymer waveguide and the optical fiber, and provide waveguide equipment which has a high reliability for moisture and temperature change.

In one embodiment of the present invention, waveguide equipment comprises a polymer waveguide having a core and cladding, and an optical fiber which is connected to an end surface of the polymer waveguide with glue and which is optically connected with the core, wherein an oxide film formed between at least one end surface of the polymer waveguide or the optical fiber and the glue.

In an aspect of the present invention, a polymer waveguide comprises a core and a cladding of the polymer waveguide being formed on a substrate made of mineral matter materials, wherein an oxide film formed between at least one surface faced each other of the substrate or the cladding, and the surface of the cladding and the substrate connected via the oxide film with glue.

In one embodiment of the present invention, a polymer waveguide comprises the polymer waveguide having a core and cladding, wherein an oxide film formed on the polymer waveguide and a metal film formed on the oxide film.

As much as possible, the above mentioned constituent elements of the present invention can be combined arbitrarily. Other aspects and advantages of the invention will be apparent from the following description and the appended claims.

BRIEF DESCRIPTION OF DRAWINGS

FIG. 1 is a schematic cross-sectional view of the prior art.

FIG. 2 is a perspective view of waveguide equipment in accordance with one embodiment of the present invention.

FIG. 3 is an exploded perspective view of the waveguide equipment shown in FIG. 2.

FIG. 4 is a perspective view of the waveguide formed an oxide film on an end surface.

FIG. 5 is a longitudinal cross-sectional view of the waveguide equipment shown in FIG. 2.

FIG. 6A and 6B are figures showing a bonding state between a SiO2 film and a glue.

FIG. 7A and 7B are figures showing a bonding state between a SiOx film and a glue.

FIG. 8 is a figure to show measured IR spectrum nearby Si-OH bonding in a sample formed of a SiO1.3 oxide film and various SiO2 oxide films.

FIG. 9 shows an attenuation rate of the optical intensity of two kinds of samples, one kind of which is formed with several kinds of silicon oxide films on an end surface and one kind of which is not formed with an oxide film (prior art), and exposed high temperature high humidity environment for about 200 hours.

FIG. 10 is a perspective view showing an embodiment of a waveguide in accordance with the present invention.

FIG. 11 is a cross-sectional view showing an embodiment of the present invention.

FIG. 12 is a cross-sectional view showing an embodiment of the present invention.

FIG. 13B is a cross-sectional view of a waveguide used for an embodiment of the present invention and FIG. 13A is a figure to show a manufacturing process of the waveguide.

FIG. 14B is a cross-sectional view of a waveguide used for an embodiment of the present invention and FIG. 14A is a figure to show a manufacturing process of the waveguide.

DETAILED DESCRIPTION

The present invention is explained specifically below with reference to specific embodiments. These embodiments are merely examples, and the present invention is not limited to only the embodiments described below.

Specific embodiments of the present invention are explained in detail below with reference to the figures. These embodiments are merely examples and the present invention is not limited to the specific embodiments explained below.

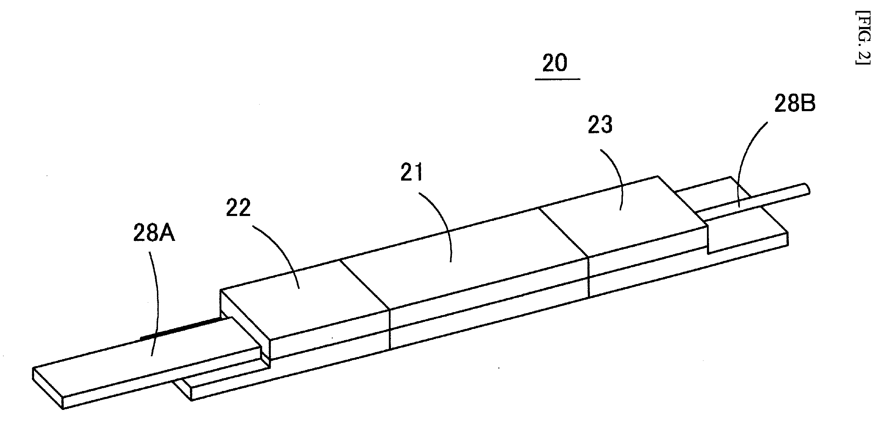

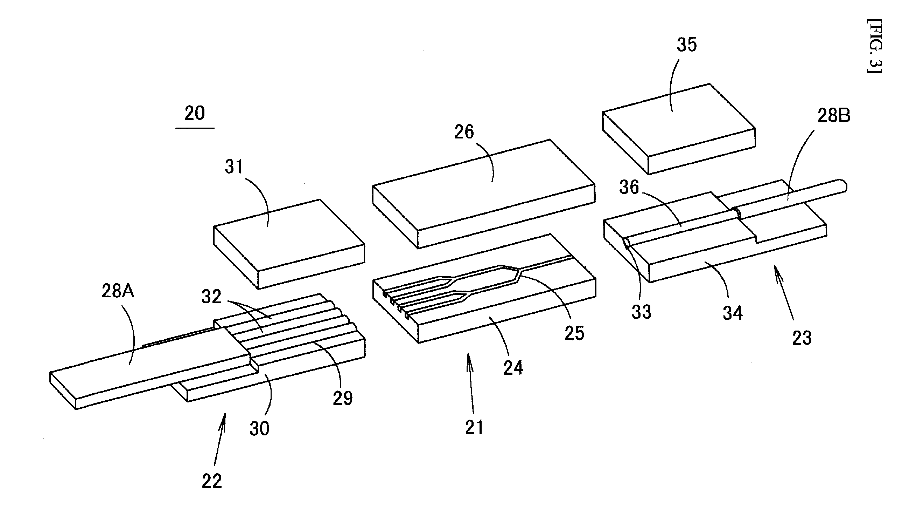

FIG. 2 is a perspective view of waveguide equipment 20 in accordance with one embodiment of the present invention. FIG. 3 is an exploded perspective view of the waveguide equipment 20. The waveguide equipment 20 is formed by a single mode waveguide 21 and fiber guides 22, 23 for I/O port connected with both sides of the waveguide 21. The waveguide 21 is formed a under cladding 24, a core 25, and an over cladding 26. The under cladding 24 is made of transparent resin of the high refractive index and has a concave groove on a part of the top surface. The core 25 is formed by burying in the concave groove transparent resin with a higher refractive index than the under cladding 24. The over cladding 26, which is made of transparent resin with a lower refractive index than the core 25, is put on the top surface of the under cladding 24. Both end sides of the core 25 are exposed at an end surface, which is put between the under cladding 24 and the over cladding 26 in the waveguide 21. In one specific embodiment, the width and height of the core 25 is about 6μm in the case of the single mode waveguide. The transparent resin of the under cladding 24 and the over cladding 26 can be different transparent resin, but it is desirable to use the same resin.

The ultraviolet radiation hardening type transparent resin is desirable as the resin to form the over and under cladding 26, 24 and the core 25, but the heat curing type transparent resin can also be used. Similarly, PMMA (polymethylmethacrylate), photo-PCB (photo-curing type polychlorinated biphenyl), alicyclic epoxy resin, photosensitive cationic polymerization initiator, acrylate resin (containing Si and F), photosensitive free-radical polymerization initiator, and fluoridated polyimide (these resins are not limited to a photo-curing type.) can be used as the transparent resin to form the over and under cladding 26, 24 and the core 25. A reproduction method with a stamper is preferable to form the under cladding 24, but hot pressing, etching, and injection molding can be used to form the under cladding 24.

A lot of the waveguide 21 is produced on a glass wafer at one time in a mass production, which improves productivity. The many optical waveguides 21 produced on the wafer are cut by dicing methods, and they are split into a piece of the waveguide 21. At this time, the end surface of each waveguide 21 is ground, and both end surfaces of the core 25 exposed may be finished smoothly.

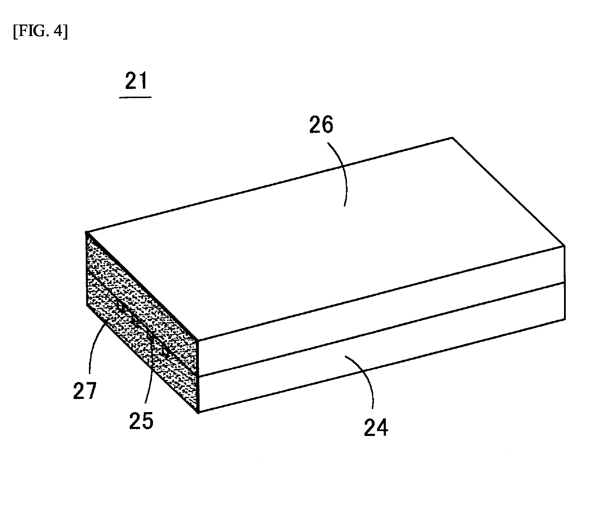

After that, as shown in FIG. 4, an oxide film 27 is layered on the entire surface of both end sides of the waveguide 21 by sputtering method, evaporation method, ordinary temperature CVD method, or photo-CVD method. For example, a $SiO_x$ film is layered on the end surface of the waveguide 21 as the oxide film 27 by sputtering method using silicon. A sputtering condition of this case uses argon plasma and $SiO_x$ having x value as desired (for example, SiO1 .3). In one specific example, the arrival pressure force is $3\times10$-6 Torr. The deposition pressure force is $5\times10$-3 Torr. The flow rate of argon is 20 sccm. The high frequency output power is 0.2 kW. The deposition time is two minutes. The film thickness of the oxide film 27 was 1000 Å.

In this production process, molecules consisting of the oxide film 27 arrive at the end surface of the waveguide 21 with kinetic energy by the sputtering method and the evaporation method. So, the molecules are bonded with more OH radicals by ionic bonding or chemical bonding at the end surface of the waveguide 21. As a result, the exfoliative strength between the waveguide 21 and the oxide film 27 can be raised as compared to glue applied on the end surface of the waveguide directly.

Silicon oxide film for the oxide film 27 is preferable, but the transparent oxide film such as even aluminum, magnesium, or SiON are preferable if the coupling efficiency between the optical fiber and the waveguide 21 does not become decreased. The oxide film in which the number of oxygen atoms is less than the most stable stoichiometric composition is desirable. For example, $SiO_x$ (x = 1 to 1.5), which has smaller oxygen content than $SiO_2$, is desirable as the silicon oxide film. The film that has a smaller ratio of an oxygen atom than $Al_2O_3$ is preferable as the aluminum oxide film.

It is desirable that the thickness of the oxide film 27 is thinner than 4000 Å. That is because it prevents degradation of an optical transmission rate in the oxide film 27 and crack outbreak by internal stress of the oxide film 27. In addition, it is desirable that the thickness of oxide film 27 is thicker than 500Å. That is because it prevents water or steam from getting into the waveguide 21 through the oxide film 27. In addition, it is necessary that the oxide film 27 be layered by a cold temperature grown method of less than 200 degrees Celsius in order to not make the waveguide 21 deteriorate because it is a deposition on the resin of the waveguide 27 that the oxide film 27 is layered. In addition, a substrate temperature in a deposition equipment should be kept not more than 100 degrees Celsius when the oxide film is layered more than 2000 Å to prevent cracks. So, the quality of the oxide film can be improved.

As shown in FIG. 3, the fiber guide 22 is made from a substrate 30, which is made of glass or plastic and is formed of plural of V-grooves 29 on a top surface and a fiber weight 31. A tape core 28A is torn off the coating of the end and exposes plural optical fibers 32 composed of a core and a cladding. Each optical fiber 32 is held in position in each V-groove 29 of the substrate 30 by the fiber weight 31 with the glue put on the optical fiber 32. Fiber weight 31 presses onto each optical fiber 32 so that the substrate 30 and the fiber weight 31 are integrated. Similarly, the fiber guide 23 is made from a substrate 34, which is made in glass or plastic and is formed of a V-groove 33 on a top surface and a fiber weight 35. A tape core 28B is torn off the coating of the end and exposes an optical fiber 36 composed of a core and a cladding. The optical fiber 36 is held in position in the V-groove 33 of the substrate 34 by the fiber weight 35 with the glue put on the optical fiber 36. Fiber weight 35 presses onto the optical fiber 36 so that the substrate 34 and the fiber weight 35 are integrated. In addition, the optical fibers 32, 36 can be preferably made of either glass fiber or plastic fiber.

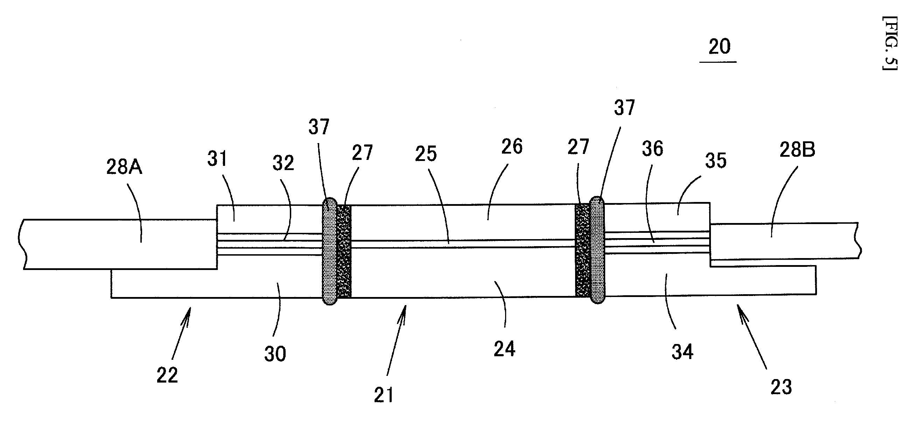

As shown in FIG. 5, after each waveguide 21 and the fiber guide 22, 23 are produced in this way, the waveguide equipment 20 is assembled so that the center of the core 25 and the central axes of the optical fiber 32, 36 are align and couple optically. FIG. 5 is a sectional view to show the waveguide equipment 20 assembled by bonding the fiber guide 22, 23 to both ends of the waveguide 21. In other words, glue 37 is applied between the oxide film 27 formed by both end sides of the waveguide 21 and fiber guide 22, 23. Thus, the waveguide 21 and the fiber guide 22, 23 are integrated with the glue 37. Primer coating may be put on the surface of oxide film 27 to improve adhesion with the oxide film 27 and the glue 37.

If the oxide film 27 is formed on the end surface of the waveguide 21, the adhesion between the oxide film 27 and the glue 37 improves and the adhesion between the oxide film 27 and the waveguide 21 also improves. So, the adhesive strength improves between the waveguide 21 and the fiber guide 22, 23. However, if the $SiO_2$ that has a stable composition is used as the oxide film 27, the exfoliative strength between the oxide film 27 and the glue 37 becomes lower and the exfoliative strength between the oxide film 27 and the waveguide 21 also becomes lower. So, the adhesive strength becomes lower between the waveguide 21 and the fiber guide 22, 23. On the other hand, if $SiO_x$ ($1 \leqq x \leqq 1.5$) that has a composition ratio of an oxygen atom which is smaller is used as the oxide film 27, the exfoliative strength between the oxide film 27 and the glue 37 can be improved and the exfoliative strength between the oxide film 27 and the waveguide 21 can be improved. Thus, the adhesive strength can be made higher between the waveguide 21 and the fiber guide 22, 23.

Secondly, the adhesive strength improves when an oxide film 27 that has a composition ratio of an oxygen atom which is smaller is formed on the end face of the waveguide 21. When $SiO_2$ is formed as the oxide film 27 on the resin surface of the waveguide 21, that resin and the oxide film 27 are bonded because the OH radicals of the resin surface of the waveguide 21 and of the oxide film 27 bond chemically. However, $SiO_2$ is a stable composition and bonding of each atom is saturated. Thus, there is less excess binding residue in $SiO_2$. $SiO_2$ and the resin bond chemically through an oxygen atom included in the OH radicals on the resin surface of waveguide 21, but the exfoliative strength is low between the waveguide 21 and the oxide film 27 for lack of binding residue with chemical bond.

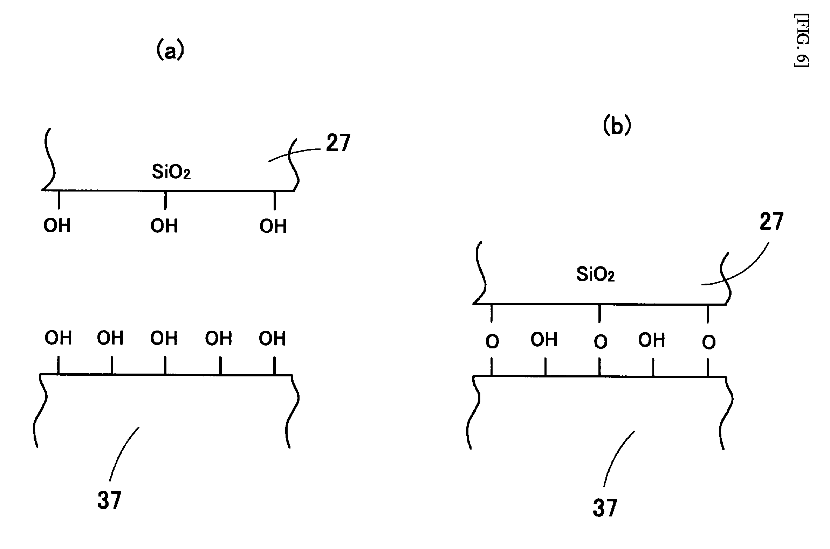

When $SiO_2$ is formed as the oxide film 27, the bonding between the glue 37 and the oxide film 27 is also the same way. As shown in FIG. 6A, there is less excess binding residue in $SiO_2$. So, as shown in FIG. 6B, there is less binding residue for chemical binding between $SiO_2$ and the glue 37 through an oxygen atom included in the OH radicals on the surface of the glue 37. Therefore, the exfoliative strength is also low between the glue 37 and the oxide film 27.

On the other hand, the bonding state is unstable for unsaturated bonding by lack of an oxygen atom

in $SiO_x$ ($1 \leqq x \leqq 1.5$). Therefore, many OH radicals appear on the surface of $SiO_x$ because an atmospheric H atom is bonded to $SiO_x$, the oxide film 27. As a result, the OH radicals in $SiO_x$ react to the OH radicals in the waveguide 21 as follows.

$$OH^- + OH^- \rightarrow O_2^- + H_2O$$

So, many OH radicals bond chemically through O atoms. Therefore, the exfoliative strength between the waveguide 21 and the oxide film 27 can be improved by using $SiO_x$ ($1 \leqq x \leqq 1.5$) as the oxide film 27.

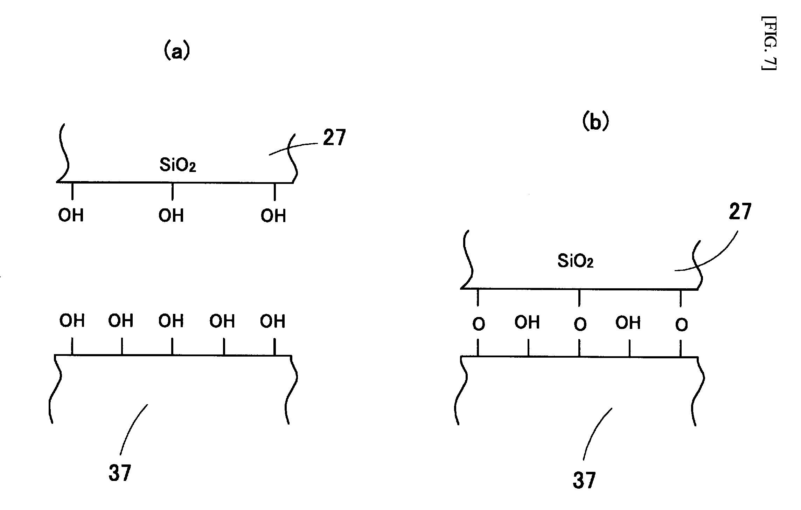

When $SiO_x$ ($1 \leqq x \leqq 1.5$) is formed as the oxide film 27, the bonding between the glue 37 and the oxide film 27 is also the same way. The bonding state of the oxide film 27 is unstable for unsaturated bonding by lack of an oxygen atom. Therefore, as shown in FIG. 7A, many OH radicals appear on the surface of $SiO_x$ because an atmospheric H atom is bonded to $SiO_x$, the oxide film 27. As shown in FIG. 7B, the OH radicals in $SiO_x$ react to the OH radicals in the glue 37 and these many OH radicals bond chemically through O atoms. So, the exfoliative strength between the glue 37 and the oxide film 27 can be improved by using $SiO_x$ ($1 \leqq x \leqq 1.5$) as the oxide film 27.

As a result, both the exfoliative strength between the oxide film 27 and the waveguide 21 and the exfoliative strength between the oxide film 27 and the glue 37 can be improved by using $SiO_x$ ($1 \leqq x \leqq 1.5$) as the oxide film 27. Furthermore, the adhesive strength between the waveguide 21 and the glue 37 can be improved.

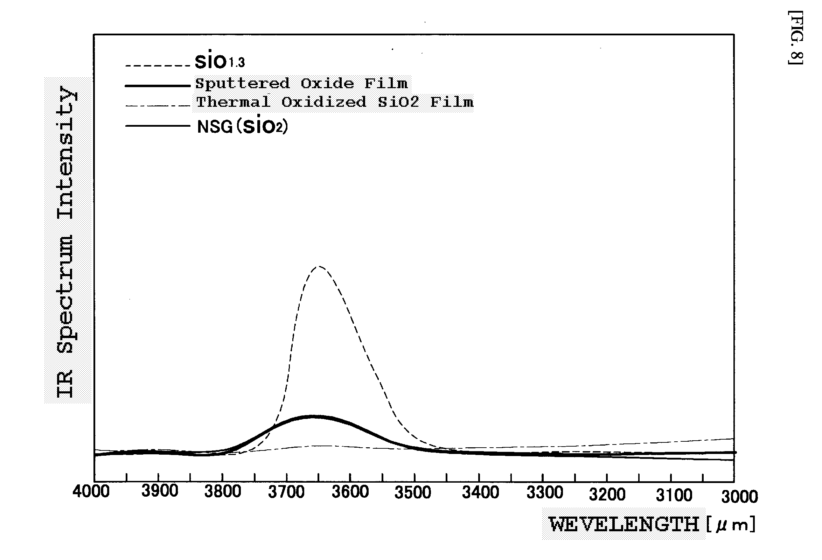

FIG. 8 shows an IR spectrum intensity nearby Si-OH bonding measured with samples of $SiO_{1.3}$, sputtered oxide film ($SiO_2$), thermal oxidized $SiO_2$ film, and NSG ($SiO_2$ by the CVD method). In this figure, a horizontal axis shows a wavelength and vertical axis shows the IR spectrum intensity. This figure also shows that the number of OH radicals increase and the exfoliative strength between the resin and the oxide film are higher as the value of the vertical axis becomes larger. As can be seen from this figure, the OH radicals hardly appear in $SiO_2$, but that several times the OH radicals appear in $SiO_{1.3}$.

In addition, it is known that internal stress of $SiO_x$ ($1 \leqq x \leqq 1.5$) film is smaller than that of $SiO_2$ film. For example, internal stress of $SiO_{1.3}$ is 1/5 compared with $SiO_2$ film. According to an experiment, $SiO_2$ film was completely damaged when a sample consisting of polymer waveguide formed on a glass substrate, $SiO_2$ film formed thereupon, and a glass substrate bonded thereupon, was exposed for 20 hours to high temperature and high humidity condition. In contrast, $SiO_{1.3}$ film was not damaged as only some wrinkles occurred to the oxide film when a sample consisting of polymer waveguide formed on a glass substrate, $SiO_{1.3}$ film formed thereupon, and a glass substrate bonded thereupon, was exposed for 20 hours to high temperature and high humidity condition. Therefore, the waveguide equipment 20 which is hard to deteriorate under high temperature and high humidity condition, superior in reliability, and having high adhesive strength can be produced using $SiO_x$ ($1 \leqq x \leqq 1.5$) as oxide film 27.

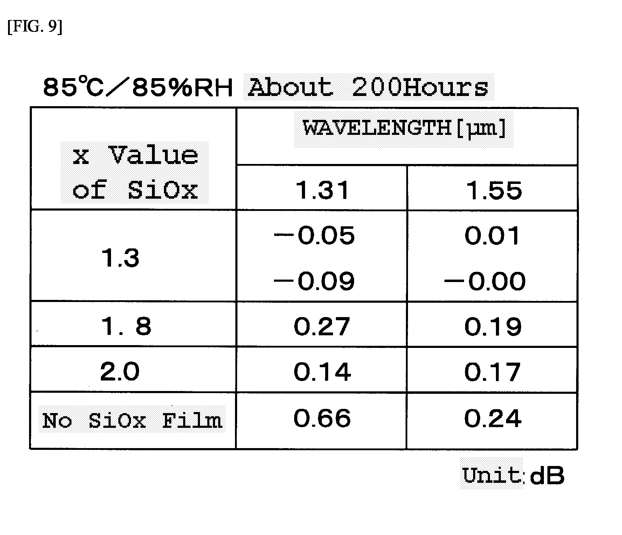

Next, two kinds of samples of the waveguide 21 in which the resin is easy to change its characteristic at high temperature and high humidity condition are presented. As shown in FIG. 9, one sample is formed silicon oxide film on the end surface of the waveguide 21 and another sample has no silicon oxide film. After these samples are exposed to a condition of high temperature (85 degrees Celsius) and high humidity (85% RH) for about 200 hours, an attenuation rate of signal strength is measured using an optical signal of 1.31μm wavelength and 1.55μm wavelength. The result is shown in FIG. 9. As can be seen from FIG. 9, the $SiO_{1.8}$ film sample, the $SiO_2$ film sample, and no oxide film sample showed large damping by deterioration. But the $SiO_{1.3}$ film sample showed very small damping.

A break down test was done with the waveguide equipment 20 produced above. The waveguide equipment 20 was put into a PCT (Pressure Cooker Testing machine) at the break down test. As a result, it is made sure that adhesive strength was kept without damaging the oxide film 27 which was formed on the end of the waveguide 21 even after more than 50 hours of testing.

In the embodiment described above, the exemplary waveguide was single mode, but a multimode waveguide that has the same structure can be made using the same production method.

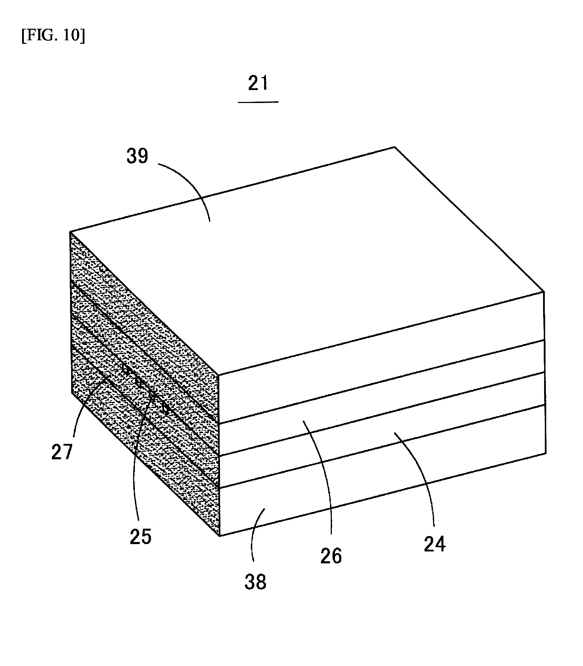

FIG. 10 is a perspective view to show an embodiment of waveguide 21. In this embodiment, the waveguide 21 comprises the under cladding 24, the core 25 and over cladding 26. But in this embodiment, as shown in FIG. 10, the waveguide 21 which has the under cladding 24, the core 25 and the over cladding 26 may be put between a bottom substrate 38 and an upper substrate 39 made of mineral matter materials. The bottom substrate 38 and the upper substrate 39 should be used as the glass substrates which are mineral matter materials. In addition, a quartz glass or an optical glass may be used as a glass substrate.

In this embodiment, the oxide film 27 is formed on the end surface of the waveguide 21 made of organic materials and mineral matter materials. The end surface of the waveguide 21 formed oxide film 27 and the fiber guide 22 and 23 are adhered with the glue 37. Even this embodiment, when the $SiO_2$ film is formed as the oxide film 27, the oxide film 27 is damaged after exposed in high temperature and high humidity condition for a long time. On the other hand, if $SiO_x$ ($1 \leqq x \leqq 1.5$) film is formed as the oxide film 27, the internal stress of oxide film 27 shrink and the oxide film 27 is not damaged after exposed in high temperature and high humidity condition for a long time.

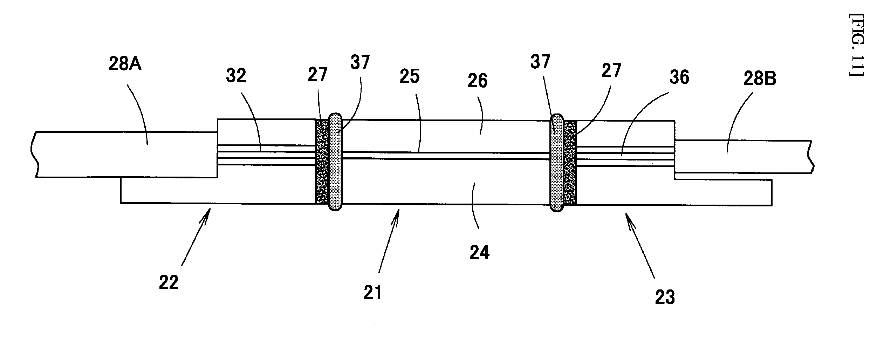

FIG. 11 is a sectional view showing an embodiment of the present embodiment. In this embodiment, the oxide film 27 is formed on an end surface of the connection side in the fiber guide 22 and 23. And the end surface of the fiber guide 22, 23 and the end surface of the waveguide 21 are adhered with the glue 37. In this embodiment, the fiber guide 22, 23 is made of plastic and the optical fiber 32, 36 is also made of plastic. So, the adhesive strength is not enough when the glue is applied directly. Thus, the adhesive strength between the waveguide 21 and the fiber guide 22, 23 can be made higher by forming the oxide film at the end of the fiber guide 22, 23 and improving the adhesion between the fiber guide 22, 23 and the glue 37. In addition, the moisture which is a factor to make the adhesive strength with the glue 37 deteriorate may invade the glue 37 through the optical fiber 32, 36. But the oxide film 27 intercepts the moisture by forming on the end surface of the fiber guide 22, 23 and sealing the end surface of the optical fiber 32, 36. Therefore, it is hard for moisture to reach the glue 37 and degradation of the adhesive strength by moisture can be prevented.

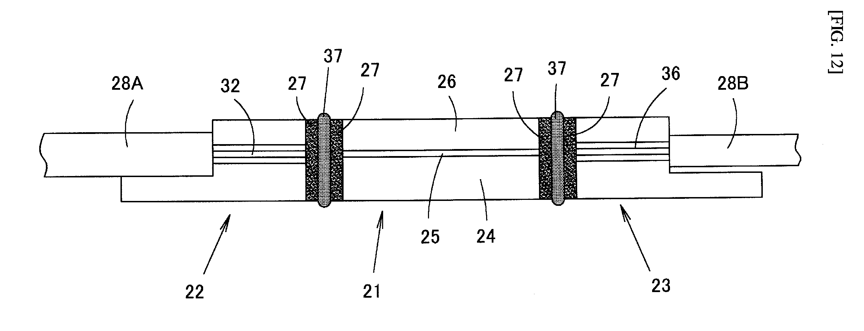

FIG. 12 is a sectional view showing an embodiment of the present embodiment. In this embodiment, the oxide film 27 is formed on both end surfaces of waveguide 21 and of the fiber guide 22, 23. And it is bonded with the glue 37 between the oxide film 27 of waveguide 21 and the oxide film 27 of the fiber guide 22, 23. Therefore, even if both the waveguide 21 and the fiber guide 22, 23 are made of plastic, the adhesive strength between the waveguide 21 and the fiber guide 22, 23 can be raised by improving the adhesion between the glue 37 and the waveguide 21, the fiber guide 22, 23.

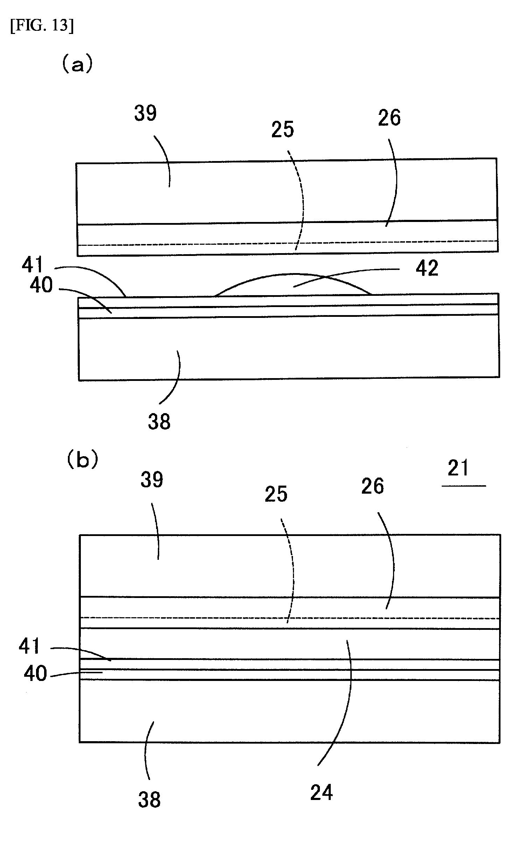

FIG. 13B is sectional view of the waveguide 21 used an embodiment of the present invention. FIG. 13A shows the manufacturing process of the waveguide 21. In the waveguide 21 ofthis embodiment, an oxide film 40 such as $SiO_x$ $(1 \leqq x \leqq 2)$ is formed on the bottom substrate 38 made of silicon and primer 41 is applied thereupon. Over cladding 26 that buries the core 25 is formed on an under surface of the upper substrate 39 made of glass. As shown in FIG. 13A, resin 42 for the under cladding is dropped on the primer 41. The resin 42 pushed from above by the upper substrate 39 is spread out between the primer 41 and the over cladding 26, and make rigid by UV radiations. Thus, the cladding 24 is formed. As shown FIG. 13B, in the waveguide 21 made in such a way, the adhesive strength between the under cladding 24 and the bottom substrate 38 can be improved because the oxide film 40 is formed. So, it is hard for exfoliation to occur between the under cladding 24 and the bottom substrate 38 under a high temperature and a high humidity condition.

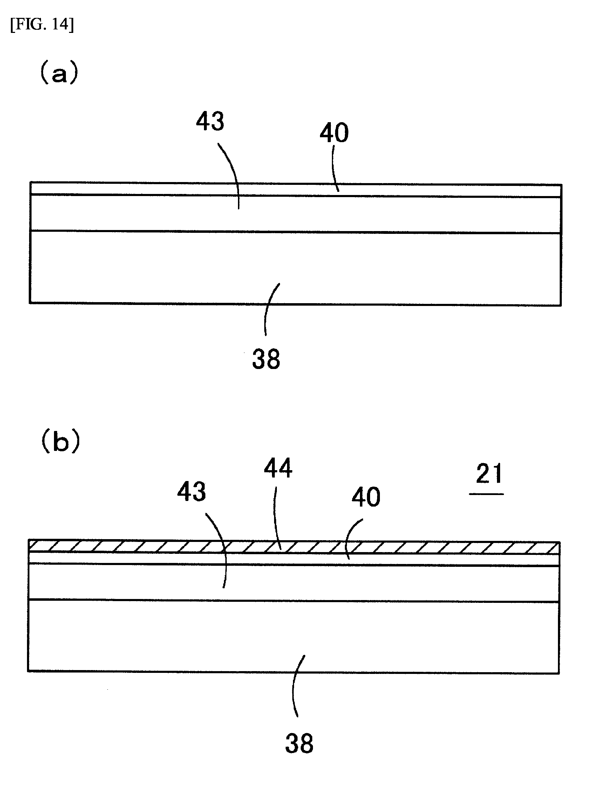

FIG. 14B is a sectional view of the waveguide 21 used in an embodiment of the present invention. FIG. 14A shows the manufacturing process of the waveguide 21. In the waveguide 21 by this embodiment, as shown in FIG. 14A, the waveguide layer 43 which comprises the under cladding 24, the core 25, and the over cladding 26 is formed on the bottom substrate 38 made of glass. And the oxide film 40 such as $SiO_x$ $(1 \leqq x \leqq 2)$ is formed on the waveguide layer 43. Next, a metal film 44 is formed on the oxide film 40 as an electrode by sputter. Thus, the waveguide 21 shown in FIG. 14B is obtained.

In the waveguide 21, as shown FIG. 14B, made in such a way, the adhesive strength between the waveguide layer 43 and the metal film 44 can be improved because the oxide film 40 is formed. So, it is hard for exfoliation of the metal film 44 to occur under a high temperature and a high humidity condition. Especially, in the metal film 44, it is easy for exfoliation of the metal film 44 to occur because chipping of the substrate and turning up of the metal film occur during the dicing process of the waveguide 21. The adhesive strength of the metal film 44 at the dicing process can be improved because the metal film 44 is formed on the oxide film 40. If the metal film 44 is used as the electrode, bonding wires may be bonded on the metal film 44. In such instances, the shearing stress can be added to the metal film 44, but the shearing strength can be done more than 5 times by forming the metal film 44 on the oxide film 40.

In the above embodiment, the waveguide equipment is coupled to an optical fiber on both sides of the waveguide. Those skilled in the art will appreciate that an optical transceiver, such as a photo detector or a light projection device, may be connected to the waveguide equipment. Also, various kinds of forms such as an optical coupler, a WDM coupler, a VOA (a variable optical attenuator), an optical switch, and a multimode waveguide device may be used.

While the invention has been described with respect to a limited number of embodiments, those skilled in the art, having benefit of this disclosure, will appreciate that other embodiments can be devised which do not depart from the scope of the invention as disclosed herein. Accordingly, the scope of the invention should be limited only by the attached claims.

## Claims

1. Waveguide equipment (20), comprising:

   a polymer waveguide (21) having a core (25) and a cladding (26), and
   an optical fiber (36) bonded to an end surface ofthe polymer waveguide with glue (37) and coupled optically with the core,

   wherein an oxide film (27) is formed between at least one end surface of the polymer waveguide or the optical fiber and the glue.

2. The waveguide equipment according to claim 1, wherein a composition of the oxide film having a ratio of oxygen atomic number smaller than a composition ratio that is stable.

3. The waveguide equipment according to claim 1, wherein materials of the oxide film are $SiO_x$ (wherein $1 \leqq x \leqq 1.5$).

4. The waveguide equipment according to claim 1, wherein
a thickness of the oxide film is within a range from 500 Å to 4000 Å.

5. The waveguide equipment according to claim 1, wherein
the core and the cladding of the polymer waveguide are formed on a substrate (38) made of mineral matter materials.

6. A polymer waveguide, comprising:

   a core (25) and a cladding (26) of the polymer waveguide formed on a substrate (38) made of mineral matter materials, wherein
   an oxide film (40) formed between at least one surface facing each other of the substrate or the cladding, and
   the surface of the cladding and the substrate are bonded with glue through the oxide film.

7. A polymer waveguide, comprising:

   a polymer waveguide having a core (25) and a cladding (26),

   wherein an oxide film (40) is formed on the polymer waveguide and a metal film (44) is formed on the oxide film.

[FIG. 1]

[FIG. 2]

[FIG. 3]

[FIG. 4]

[FIG. 5]

[FIG. 6]

EP 1 569 018 A2

(a)

SiO₂ — 27

OH        OH        OH

OH    OH    OH    OH    OH

37

(b)

SiO₂  27

O    OH    O    OH    O

37

13

[FIG. 7]

[FIG. 8]

[FIG. 9]

## 85°C／85%RH About 200Hours

| x Value of SiOx | WAVELENGTH [μm] | |
|---|---|---|
| | 1.31 | 1.55 |
| 1.3 | −0.05<br>−0.09 | 0.01<br>−0.00 |
| 1. 8 | 0.27 | 0.19 |
| 2.0 | 0.14 | 0.17 |
| No SiOx Film | 0.66 | 0.24 |

Unit:dB

[FIG. 10]

21

39

27

25

38

26

24

[FIG. 11]

[FIG. 12]

[FIG. 13]

(a)

(b)

[FIG. 14]

(a)

(b)